# EUROPEAN PATENT APPLICATION

(11) **EP 1 023 910 A1**
(43) Date of publication of application: **02.08.2000**
(21) Application number: 99810078.8
(22) Date of filing: 29.01.1999
(51) Int. Cl.: A61L 27/00, A61L 31/00

(54) **Preparation of osteophilic surfaces for metallic prosthetic devices anchorable to bone**

(71) Applicant: Institut Straumann AG, 4437 Waldenburg (CH)
(72) Inventor: Steinemann, Samuel Prof., 1025 Saint-Sulpice (CH); Simpson, James Dr., 4458 Eptingen (CH)
(74) Representative: Braun, André

(57) **Abstract**

A chemical factor contributes above all to the integration in bone of a titanium prosthetic device. The prosthetic device has hydrophilic properties and it has a large effective surface area which is conditioned by a suitable electrolyte comprising mono- and two-valent metals.

## Description

### Technical field

The invention relates to titanium or titanium base prosthetic devices meant to be implanted into the skeletal system by direct bone apposition to the device surface and essentially to their surface preparation and chemical processes which can enhance the bonding between device and bone.

### Background art

The traditional methods for fixation of a prosthetic device to the skeletal system fall into several categories. These include mechanical fixation to bone using nails or screws, impaction of the device into hard tissue, and the use of bone cement, e.g. polymethylmethacrylate, as a grouting agent between the endoprosthesis and bone. New alternate methods comprise fixation by bone ingrowth into porous surfaced devices, by chemical bonding between bone and surface-active ceramic devices, e.g. having a hydroxyapatite coating, and by direct bone apposition to the surface of a titanium-base metal device. As to the latter form of fixation, Brånemark has coined the term *osseointegration* and defines it as *direct structural and functional connection between ordered, living bone and the surface of a load-carrying implant.*

The structural and functional connection between bone and the device can be achieved by fixation rationales of (a) macrointerlock, e.g. devices with large threads or grooves cut into the surface or with macroporous sintered bead coatings, and (b) microinterlock, e.g. devices with special surface textures created by plasma-spraying or grit-blasting or chemical etch. The anchorage of such devices to bone can be assessed by mechanical measurements such as shear tests (push-out and release torque) or tensile tests (tear-off) and by measurements of the bone-device contact area in histological preparations. Many experiments indicate that these properties are connected with the surface texture of the device, in particular with its roughness (see review by Brunski, Clinical Materials, Vol. 10, 1992, pp. 153-201).

Fracture healing processes are cell mediated and same mechanisms are active for osseointegration of a titanium-based prosthetic device. Surface characteristics can influence the needed cellular activity. Considering surface texture, in vitro experiments with human osteoblast-like cells indicate that surface roughness alters cell proliferation, differentiation, and matrix mass production. Further, it is expected that roughness plays a role in determining phenotypic expression of cells *in vivo,* i.e. the observable characteristics of cells and tissue differ from those of its genotype (Boyan et al., Biomaterials, Vol. 17, 1996, pp. 137-146).

Rough surfaces are applied to prosthetic devices with the concept that this would aid cell adhesion. But the question of the reaction of cells to surface roughness is an intriguing one. There are problems at the simple level of defining roughness and there are difficulties for the quantification of this roughness. No work has yet discovered convincing rules for roughness height and roughness spacing of a surface topography to which a bone cell will rapidly and efficiently react (see review by Curtis and Wilkinson, Biomaterials, Vol. 18, 1997, pp. 1573-1583).

The question of whether the bone cells are reacting to the topography directly or to a patterned substratum chemistry formed on the topography must be raised in view of Brånemark's definition of osseointegration. The wording *functional connection* suggests to consider factors beyond surface texture, in particular surface area and surface chemistry, which truly determine the size of bonding forces between the living bone and the load-carrying device. These surface properties of the metallic device clearly depend on the mechanical and chemical preparation techniques used.

Surface analyses, by X-ray photoelectron spectroscopy and Auger electron spectroscopy, of clinically used implants prior to insertion indicate a contamination. In fact, customary surface preparations of titanium and its alloys for laboratory and clinical work inevitably leave some contamination consisting essentially of carbon compounds and small or trace amounts of N, Ca, S, P, Si. These contaminations accumulate in the outermost volume of the surface oxide. An admissible concentration of C, measured by XPS, AES or other surface sensitive spectroscopic methods, is 20 atom% in order to maintain the hydrophilic (water attracting) character of the implant. Contamination also influences the wetting behaviour of the surface. Hydrocarbon adsorption beyond the above limit give water contact angles greater than 60°, i.e. hydrophobic (water repelling) character of the surface. Inadequate cleaning and sterilization procedures are the cause of an adsorption of inorganic and organic matter. Such surfaces are biologically passive.

When inserted, a prosthetic device will interact with tissue and its surface properties will have an enormous effect on the rate, the extent, and the mechanism of adsorption of tissue fluid components and in particular of proteins (see review by Brash and Horbett, in Proteins at Interfaces II, Horbett and Brash eds., ACS Symp. 602, Amer. Chem. Soc. Washington 1995, pp. 1-23). A widely accepted generalization regarding this influence holds that the more hydrophobic the surface the greater the extent of adsorption, and that adsorption on hydrophobic surfaces is essentially nonspecific for the macromolecule.

It follows from these considerations that the problems of proper handling of prosthetic devices until the time of implantation, in order to obtain beneficial interactions between the surfaces of the prosthetic devices and the bone, have yet not been addressed properly and that there is still a need for prosthetic devices which exhibit good osseointegration when implanted into bone.

### Disclosure of the invention

According to the invention titanium or titanium base alloy prosthetic devices are provided which have at least one surface for attachment of bone, said devices being enclosed in a container and being characterised in that the said surface is hydrophilic and hydroxylated, when the device is removed from the container.

The applicants have found that surfaces can be rendered biologically active and that specific protein-surface interaction involving chemical functional groups can occur only when contamination is avoided and when the surface has hydrophilic character and is hydroxylated.

The applicants have further found that the biological effectiveness of the surface can be maintained by conditioning the surface with suitable electrolytes and also enhanced by increasing the effective surface area.

### Ways of carrying out the invention and advantageous effects of the invention

Examples for the devices of the present invention are dental implants and artificial joints.

According to the invention, the chemical factor which contributes to the osseointegration of titanium based prosthetic devices is the hydroxylated and hydrophilic character of the metal oxide surface and the large effective area of this surface. The large surface area is prepared by mechanical, chemical or other means and the biologically active state of the oxide surface is conditioned by suitable electrolytes and eventually by grafting with two-valent cations.

Titanium is a reactive metal. In air and aqueous electrolytes, an oxide is spontaneously formed on the surface of the metal. In the case of a prosthetic device, the outer surface of this oxide interacts with living matter.

Most metal oxides are hydroxylated when water or its vapor has had access to the surface. The surface hydroxydes are the functional groups in any interaction with tissue fluids, proteins and cells. In the case of titanium oxide, the density of functional groups is 4 to 5 per nm² and these hydroxyls confer to the surface a high charge which is positive for pH smaller than about 6 and negative for a pH larger than about 6 of the electrolyte (Boehm, Discussions Faraday Society, Vol. 52, 1971, pp. 264-275). The pH value for charge reversal is called point-of-zero-charge (PZC). The character of the surface oxide on titanium based metals is thus amphoteric and hydrophilic. The pseudobiological activity of the titanium surface expresses these unique chemical properties.

One advantageous effect of the invention is to augment the phenotypic expression of the titanium based devices by increasing the number of functional groups through a larger effective surface area.

The currently prevailing view that roughness determines the strength of osseointegration is void; texture as described by roughness is not more than an apparent parameter and can at best give a hint why a rough surface may favor the anchorage of bone. The real parameter for biological activity of a surface, i.e. bonding to tissue, is chemical affinity and surface charge which are proportional to surface area. It is also noted that no immediate or analytical relation between roughness and effective surface area exists.

Several methods for measuring the surface area of a metal can be used: (a) electrokinetic experiments, in particular measurements of electrophoretic mobility, (b) surface charge determination by acid-base titration, (c) impedance spectroscopy, and (d) voltammetry. In method (c), the electrical impedance of a sample of known dimensions exposed to a neutral electrolyte is determined as a function of frequency and capacity and polarization resistances are extracted. The surface area is proportional to capacity. In method (d), a voltage sweep is applied and the current is measured; an oxide film will grow on titanium and the consumed current is proportional to the total surface. For all these measurements, the reference is a flat polished sample and the ratio of charges, capacities or currents gives a number for the surface area enhancement produced by the specific surface treatment.

Some examples of surface preparations and their effect on surface area enhancement are given in the following table 1:

| **surface treatment** | **method of measurement** | **electrolyte** | **surface area enhancement** |
|---|---|---|---|
| polished/etched | impedance | 0.1M Na₂SO₄ | 1.9 |
| sandblasted/etched | impedance | 0.1M Na₂SO₄ | 3.9 |
| plasma sprayed | impedance | 0.1M Na₂SO₄ | 15 |
| coarse sandblasted | voltammetry | 0.15 M NaCl | 5.8 |
| sandblasted/etched | voltammetry | 0.15 M NaCl | 3.6 |
| plasma sprayed | voltammetry | 0.15 M NaCl | 12 |

Since in both these two methods the surface area enhancement is obtained as the ratio between measured total surface of a test sample and the measured total surface of a flat polished reference sample both methods give essentially the same results, when used on identical test samples.

The titanium plasma sprayed surface of current technology is added for comparison; this coating has a high ratio of total to projected surface area but is porous in the mass. The surface area enhancement is manifestly not related to the classical notion of roughness and surface texture, as measured with stylus and light-optical devices.

In vitro bone cell experiments and (in vivo) histomorphometry indicate osteophilic (bone seeking) properties of the metal with its native oxide whenever the surface is uneven in small scale and when the area enhancement equals a factor of 2 or more. The processes to achieve the large effective surface area are mechanical, e.g. machining, shot peening, sandblasting, and chemical, by acid etching or electrochemical and gas plasma discharge microforming, or a combination of both ways.

Various chemical preparation methods are used for titanium prosthetic devices in laboratory work and in clinical use. Reactions do occur which change drastically the surface properties. Alcohols react with surface hydroxide groups in a condensation reaction to form esters: ≡TiOH + CH₃OH → ≡TiOCH₃ + H₂O (case of methyl alcohol; ≡Ti- is the surface metal ion). Dissolved carbon dioxide reacts by forming a bicarbonate surface complex. Carboxylic acids react with the hydroxylated surface as a conjugate (related Brønsted) base which replaces the OH-group and probably makes a covalent bond with the oxide substrate. Soluble phosphorus compounds form surface chelates (ring compounds) with metal oxides. These surface reactions are many and they are not confined to small inorganic and organic molecules. Larger molecules also, such as amino acids and proteins make surface complexes by covalent links, electrostatic interaction or hydrogen bonds. Any of these reactions can block, and inactivate the functional hydroxyl groups of the surface oxide on titanium. The hydrophilic character, connected to the presence of hydroxyls on the oxidized titanium, is lost. These are unwanted reactions because the device surface is rendered non-reactive and basically nonspecific for the adsorbent. In other words, such a surface is passive and inert.

Another advantageous effect of the invention is to provide means and ways to create the hydrophilic character and bonding affinity of the oxide interface on the titanium based devices and to preserve these surface properties up to insertion of the devices.

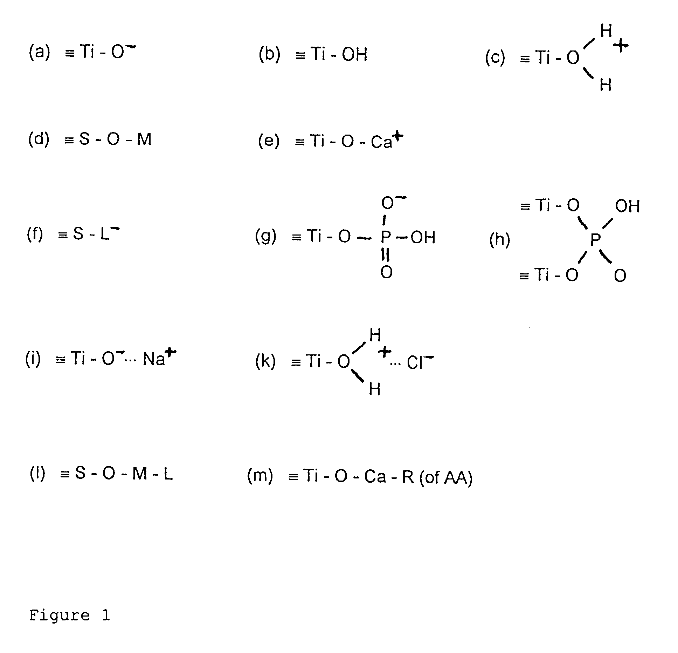

The wanted interactions at the titanium device surface are of the hydrophilic kind and the functional group for such a reaction are the surface hydroxides. Schemes and applications for the various interactions of this functional group are shown in figure 1 (Boehm, loc. cit.; Morel and Hering, Principles and Applications of aquatic Chemistry, John Wiley Sons New York, 1993, pp. 519-535).

A commonly accepted symbolism is to represent surface groups of metal oxides by a hydrolyzed species ≡SOH, where S is Ti in the present case. The gain or loss of a proton H⁺ at the surface group is considered for acid-base reactions; going from (a) to (b) and going from (b) to (c) in the table corresponds to protonation, and the reverse to deprotonation, of the surface site. Metal oxides are observed to adsorb both metals and (generalized) ligands. Replacing H⁺ by a metal provides the symbolism for cation adsorption; (d) stands for any reaction of the type and (e) is the example of calcium binding on titanium oxide. The ligand adsorption is represented by the exchange with a hydroxyl group as illustrated in (f). For (g) and (h), the double charged phosphate, which exists in an electrolyte for pH larger than about 7, replaces the hydroxyl in a monodentate and bidentate bond; the charges of the two phosphate surface complexes are different. The ligand in (f) may also be an organic molecule. Another kind of surface complex is the ion pair formation with major components of the electrolyte depicted in (i) and (k). Since the coordination space of a bound metal is only partially occupied by the surface ligands, further inorganic and organic ligands may be aquired to form a ternary complex. The general case for this complex is represented in (l). In scheme (m), a calcium atom acts as bridge in a ternary complex involving the acid residue R of an amino acid (AA). All these reactions suggest various possible coordination species that may form between solutes and an oxide surface. Evidence for the existence of a particular surface species is not given, but "reasonable" stoichiometries are chosen to fit available adsorption data.

The optimized surface should be roughened and activated. The following procedures are examples for surface preparations of devices to obtain the hydrophilic character:
- The device is shot peened and etched in dilute hydrofluoric acid at ambient temperature, or
- the device is sand blasted and etched in hot hydrochloric acid/sulfuric acid solution, or
- the device is sand blasted with coarse grit and subjected to a chemical treatment in chloric acid and/or nitric acid, or
- the device is subjected to a chemical activation treatment in a mixture of hydrochloric acid and hydrogen peroxide (proportion about 1:1:5 parts water), then washed.

Some of these surface preparations are known *per se* in the art, e.g. from EP-B-0 388 576. In the preparation of the devices of the present invention alcohols, acetone and other organic compounds are precluded in final procedures of cleaning.

In this state, the surface is chemically clean and the complexes depicted as (a), (b) and (c) in figure 1 have high reactivity for specific adsorption of metals and inorganic or organic molecules. This condition must be preserved until insertion of the device. In fact, the surface hydroxyls on the native oxide of titanium are the carriers for the direct coupling with bone mineral and with organic bone matrix.

In order to prevent contamination the devices may be kept in water having a resistivity not less than 2 Mohmcm and a total organic carbon (TOC) content of not more than 10 ppb. Another way of storage is to keep the devices in vacuum or in a protective atmosphere of inert gas such as nitrogen or argon.

The devices or the present invention are enclosed in a container such as a vial or an ampoul, in order to preserve the desired hydrophilic and hydroxylated surface. Preferably the enclosing in the container is gas-tight. In this container the surface properties are preserved until the first removal of the device from the container.

The wetting properties of a solid substrate and in particular its hydrophilicity are conveniently characterized by measurement of the contact angle between the liquid and solid in an optical set-up. The sample is dry for the measurement; if not, it is washed in pure water, then dried in a jet of nitrogen or argon gas. A drop of clean water at neutral pH is placed on the horizontally placed substrate. Adding more liquid to the drop, e.g. by using a syringe, will increase the periphery of the drop and give the advancing contact angle. On the other hand, when withdrawing part of the liquid from the drop, the receding contact angle is obtained. So as to ascertain hydrophilic character of the substrate, the contact angles for a preferred embodiment may be smaller than 45° in advancing mode or less than 15° in receding mode.

At insertion, the device will come in contact with tissue fluids comprising dissolved metals and inorganic salts such as bicarbonate, phosphate, sulfate, and also organic acids and proteins. These moieties may react with the surface hydroxide and will likely block the functional groups necessary for specific interactions with tissue.

A further advantageous effect of the invention is to provide means and ways to protect the functional surface groups and even to mimic an adhesion affinity, thus promoting cell adhesion and accelerating the integration of the device. In short, the device has osteophilic properties at insertion.

One way to protect the hydroxyl groups, i.e. to preserve a dormant and later useful property, is to keep the device in a solution of simple salts such as NaCl, KCl, NaClO₃, KNO₃, NaHCO₃, NaH₂PO₄. A concentration near physiological values and a pH value between 4 and 9 is appropriate for the biological environment. The adsorption of the singly charged cations and anions, probably by ion pair formation (figure 1, schemes (i), (k)), is weak and , if inserted in tissue, they can be desorbed and exchanged for other metals and ligands.

Another way to protect the hydroxyl groups, i.e. to preserve a dormant and later useful property, is to keep the device in a solution of divalent ions. In fact, the tendency to become adsorbed on titanium oxide is much higher for these cations. In figure 1, the metal binding is referred to as removal of a proton (deprotonation, or reaction (b) to (a)) and its replacement by a doubly charged cation (scheme (d) and (e)). The "dangling bond", after deprotonation, has a single negative charge but the surface complex, after capture of the divalent cation, has a positive charge; the adsorption is superequivalent and a surface charge reversal occurs. Any follow reaction for a ternary surface complex (scheme (l) and (m) in figure 1) must become selective. The divalent metals Mg, Ca, Sr and Mn(II) are known for their structure-building and cell-binding capabilities (see review by Hynes and Lander, Cell, vol. 68, 1992, pp. 303-322). The same metals adsorb and bind to titanium oxide for pH values higher than 6. Thus, they can occupy and protect the surface hydroxyls and they can further activate the ligand-binding functions of the extracellular matrix. The appropriate electrolyte for preparation of the device surfaces is a mixture of chlorides, chlorates, nitrates of monovalent and divalent metals of a total concentration around 150 mEq/l having a pH of 6 to 9. The unit Eq/l is the equivalent weight or combining weight concentration defined as the atomic or formula weight divided by its valence.

In both these ways the device may be packaged into the container together with the electrolyte solution which acts as a storage medium for the device in such a way that at least the surfaces that are meant to come into contact with bone are covered by the medium. The suitable container (e.g. a vial or ampoul) for the device with its storage medium is made from glass, metal or an other stable and impermeable material, and it is sealed.

## Claims

1. A titanium or titanium base alloy prosthetic device with at least one surface for attachment of bone, said device being enclosed in a sealed container, and said device being characterised in that at the time of removal of the device from the container said surfaces are hydroxylated and hydrophilic.

2. A device as in claim 1, wherein the surfaces for attachment of bone on said device have a hydrophilic surface exhibiting a contact angle in advancing mode of 45° or less in washed and dried state.

3. A device as in claim 1 or 2, wherein said container is filled with pure water having a resistivity higher than 2 Mohmcm and a total organic carbon (TOC) less than 10 ppb, said water covering the said surfaces.

4. A device as in claim 1 or 2, wherein said container contains a solution of any combination of monovalent ions in water and a total organic carbon (TOC) less than 10 ppb, for instance sodium chloride, potassium chloride, sodium nitrate, said solution covering the said surfaces.

5. A device as in claim 4, wherein the total monovalent ion concentration of said solution is in the range of 100 -200 mEq/l.

6. A device as in claim 1 or 2, wherein the container contains a solution of divalent cations of calcium, strontium, magnesium or manganese either alone or in combination with other ions in water and total organic carbon (TOC) less than 10 ppb, said solution covering the said surfaces.

7. A device as in claim 6, wherein the total ion concentration of said solution is in the range 100 -200 mEq/l and within this the divalent cation concentration in the range 1-20 mEq/l.

8. A device as in anyone of claims 1 to 7, wherein the surfaces for attachment of bone are an amphoteric oxide with point-of-zero-charge (PZC) between pH 5 and 8.

9. A device as in anyone of claims 1 to 8, wherein the surface for attachment of bone has an enhanced surface area by a factor of 2 or more, attained by machining, shot blasting or similar methods.

10. A device as in anyone of claims 1 to 8, wherein the surface for attachment of bone has an enhanced surface area by a factor of 2 or more, attained by chemical treatment methods such as etching, pickling or electrochemical attack.

11. A device as in anyone of claims 1 to 8, wherein the surface for attachment of bone has an enhanced surface area by a factor of 2 or more, attained by vacuum techniques such as plasma discharge processes or ion bombardment.

12. A device as in anyone of claims 1 to 8, the surface for attachment of bone attained by any combination of the methods given in claims 9, 10 and 11.
